# EUROPEAN PATENT APPLICATION

(11) **EP 4 317 419 A1**
(43) Date of publication of application: **07.02.2024**
(21) Application number: 22781035.5
(22) Date of filing: 30.03.2022
(51) Int. Cl.: C12N 5/077, C12N 5/0735, C12N 5/10

(54) **METHOD FOR PRODUCING CARDIOMYOCYTE MASSES**

(30) Priority: 31.03.2021 JP 2021059860
(71) Applicant: Kaneka Corporation, Osaka-shi, Osaka 530-8288 (JP)
(72) Inventor: KAWASHIMA Terasu, Kobe-shi, Hyogo 650-0047 (JP); KAWAI Yoshikazu, Kobe-shi, Hyogo 650-0047 (JP); KAMBAYASHI Sho, Kobe-shi, Hyogo 650-0047 (JP)
(74) Representative: Hoffmann Eitle
(86) International application number: PCT/JP2022/015780
(87) International publication number: WO 2022/210824

(57) **Abstract**

An object of the present invention is to easily obtain a large number of cardiomyocyte spheroids from cardiomyocytes separated into single cells. The present invention provides a method of producing a cardiomyocyte spheroid, the method comprising a step of culturing cardiomyocytes separated into single cells while stirring liquid in a container in which the cardiomyocytes are suspended, thereby causing aggregation of the cells.

## Description

### Technical Field

The present invention relates to a method of producing a cardiomyocyte spheroid.

### Background Art

Transplantation of cardiomyocytes has been proposed as methods of treating serious cardiac diseases. The forms of the cardiomyocytes to be transplanted include sheet forms and cardiomyocyte spheroid forms. Especially, cardiomyocyte spheroids can be directly injected into the heart through an injection needle. Therefore, they are considered to have a high therapeutic effect. Because a large number of cardiomyocyte spheroids are needed for such transplantation, establishment of a technique for mass-producing cardiomyocyte spheroids has been expected. As methods of producing cardiomyocyte spheroids, methods of preparing cardiomyocyte spheroids from purified cardiomyocytes separated into single cells have been known. As specific methods thereof, Patent Literature 1 and Non Patent Literature 1 report a method in which a liquid obtained by suspending cardiomyocytes in a medium is injected into a 96 round-bottom well plate and subjected to static culture, and a method in which the liquid is injected into a culture plate having a large number of microwells on a bottom surface and subjected to static culture, respectively. However, the methods are unsuitable for production methods for obtaining cardiomyocyte spheroids in an amount necessary for therapy.

### Citation List

### Patent Literature

Patent Literature 1: WO2009/017254

### Non Patent Literature

Non Patent Literature 1: Tabei R. et al., J Heart Lung Transplant. 2019; 38 (2): 203-214

### Summary of Invention

### Technical Problem

An object of the present invention is to easily obtain a large number of beating cardiomyocyte spheroids from cardiomyocytes separated into single cells.

### Solution to Problem

The above-described problems could be solved by the present invention described below.
(1) A method of producing a cardiomyocyte spheroid, the method comprising a step of culturing cardiomyocytes separated into single cells while stirring liquid in a container in which the cardiomyocytes are suspended, thereby causing aggregation of the cells.
(2) The method of producing the cardiomyocyte spheroid according to (1), wherein the container has a cell non-adherent inner face.
(3) The method of producing the cardiomyocyte spheroid according to (1) or (2), wherein a volume of the container is 5 mL or more.
(4) The method of producing the cardiomyocyte spheroid according to any of (1) to (3), wherein a speed of the stirring is changed in a stepwise or continuous manner during the culture.
(5) The method of producing the cardiomyocyte spheroid according to any of (1) to (4), wherein the speed of the stirring is increased in a stepwise or continuous manner during the culture.
(6) The method of producing the cardiomyocyte spheroid according to any of (1) to (5), wherein the cardiomyocytes are derived from a pluripotent stem cell.
(7) The method of producing the cardiomyocyte spheroid according to any of (1) to (6), wherein debris derived from the cardiomyocytes is removed in advance using a cell strainer prior to start of the culture of the cardiomyocytes.
(8) The method of producing the cardiomyocyte spheroid according to any of (1) to (7), wherein a cell concentration of the cardiomyocytes in the liquid is 1.0 × 10³ cells/mL or more and 1.0 × 10⁸ cells/mL or less.
(9) A cardiomyocyte spheroid produced by the method according to any of (1) to (8), wherein an average value of an aspect ratio is 0.60 or more.
(10) A cardiomyocyte spheroid produced by the method according to any of (1) to (8), wherein a standard deviation of a size of the cell spheroid is 50% or less of an average value of a size of the cell spheroid.
(11) A cardiomyocyte spheroid produced by the method according to any of (1) to (8), wherein an average value of a size of the cell spheroid is 30 µm or more and 2000 µm or less.
(12) A cardiomyocyte spheroid produced by the method according to any of (1) to (8), wherein the cardiomyocyte spheroid beats.
(13) A cardiomyocyte spheroid produced by the method according to any of (1) to (8), wherein a total number of cardiomyocytes constituting the cardiomyocyte spheroid is 15% or more of a number of viable cells seeded in the container.
(14) A cardiomyocyte spheroid produced by the method according to any of (1) to (8), wherein a number of a cell stained with an apoptotic marker in a cross section passing through a center of gravity of the cardiomyocyte spheroid is 50% or less of a total number of cells in the same cross section.
(15) A cardiomyocyte spheroid produced by the method according to any of (1) to (8), wherein in a case where the cardiomyocyte spheroid is double-stained with Calcein-AM and EthD-III, an area of a region stained with the EthD-III is 30% or less of an area of a region stained with the Calcein-AM.

The present specification encompasses the contents disclosed in Japanese Patent Application No. 2021-059860 on which the priority of the present application is based.

### Advantageous Effects of Invention

A large number of cardiomyocyte spheroids can be easily obtained by the method of the present invention. The present invention can contribute to widespread use of transplantation of cardiomyocytes.

### Brief Description of Drawings

[Figure 1] Figure 1 is an image showing the states of cardiomyocyte spheroids at the time of changing a blade tip speed, in Examples 1 to 3.
[Figure 2] Figure 2 is an image showing the states of cardiomyocyte spheroids 3 hours after the start of the culture, in Examples 4 and 5.
[Figure 3] Figure 3 is an image showing the states of cardiomyocyte spheroids 48 hours after the start of the culture, in Comparative Examples 1 to 3.
[Figure 4] Figure 4 is an image showing the states of the cardiomyocyte spheroids 48 hours after the start of the culture, in Examples 1 to 5.
[Figure 5] Figure 5 is an image showing the state of cardiomyocyte spheroids 24 hours after the start of the culture, in Example 6.
[Figure 6] Figure 6 shows changes in blade tip speeds during the culture, in Examples 1 to 6.
[Figure 7] Figure 7 is an image showing the Live/Dead assay results of the cardiomyocyte spheroids 48 hours after the start of the culture, in Example 4.

### Description of Embodiments

The present invention provides a method of producing cardiomyocyte spheroids, the method comprising a step of culturing cardiomyocytes separated into single cells while stirring liquid in a container in which the cardiomyocytes are suspended, thereby causing aggregation of the cells. The stirring can be performed in, for example, a container equipped with a stirring blade or a stirring bar (hereinafter also referred to as "stirring blade or the like"). The culture can be performed while performing the stirring with, for example, the stirring blade or the like. Using the above method, a large number of cardiomyocyte spheroids can be easily obtained in a short term, which is expected to contribute to widespread use of transplantation of cardiomyocytes.

### (Cardiomyocytes To Be Used)

Cardiomyocytes used in the present invention will now be described. In the present invention, cardiomyocytes separated into single cells are used in production of a cardiomyocyte spheroid. The cardiomyocytes used in the present invention are not limited but may be, for example, cardiomyocytes collected from a living body, or cells obtained by purifying and/or proliferating the cardiomyocytes, or may be cardiomyocytes induced from a stem cell or the like. The cardiomyocytes used in the present invention are preferably cardiomyocytes derived from a pluripotent stem cell, for example, cardiomyocytes obtained by differentiation induction from a pluripotent stem cell, as described later. Without limitation, a conventionally known method can be used for separating the cardiomyocytes into single cells.

The cardiomyocytes separated into single cells are preferably purified. Without limitation, a conventionally well-known method can be used for purifying the cardiomyocytes. Examples of methods of purifying the cardiomyocytes include a method using a medium having a composition in which only cardiomyocytes are viable, a method in which culture temperature is increased, and a method in which a substance that specifically acts on a pluripotent stem cell is added.

The purity of the cardiomyocytes used in the present invention is, for example, without limitation, 50% or more, 80% or more, 90% or more, or 95% or more.

Examples of indicators of the purity of the cardiomyocytes may include a cardiac troponin T positive cell rate. A cardiomyocyte spheroid having high quality is obtained in a case where the cardiac troponin T positive cell rate in the cardiomyocytes separated into single cells used in the present invention is 50% or more. Therefore, the cardiac troponin T positive cell rate in the cardiomyocytes separated into single cells used in the present invention is preferably 50% of or more, more preferably 80% or more, particularly preferably 90% or more, and most preferably 95% or more. The cardiac troponin T positive cell rate can be determined by, for example, a conventionally known immunological technique such as a flow cytometry technique with fluorescent labelling, or electrochemical luminescence immunoassay (ECLIA). When using cardiomyocytes obtained by differentiation induction from a stem cell in the present invention, the cardiomyocytes preferably exhibit the above-described cardiac troponin T positive cell rate by 15 days after the start of the differentiation induction.

The cardiomyocytes used in the present invention are more preferably ventricular muscle cells. Examples of markers of the ventricular muscle include MLC2v.

A large number of the above-described cardiomyocytes are required for cardiomyocytes transplantation therapy. A large number of cells as a source are required for performing differentiation induction to obtain the cardiomyocytes. A pluripotent stem cell such as an induced pluripotent stem cell (iPS cell) or an embryonic stem cell (ES cell) has a property of being capable of infinitely proliferating. Therefore, such a pluripotent stem cell is suitable for a source of the cardiomyocytes used in the present invention, and a nonneoplastic pluripotent stem cell such as a Muse cell is also preferred from the viewpoint of safety. In a case where the cardiomyocyte spheroid is used in therapy on a human, the cardiomyocytes are preferably derived from a human pluripotent stem cell. A long period of time can be needed before transplantation of a cardiomyocyte spheroid, when a preparation of cardiomyocytes used is started from proliferation of a pluripotent stem cell. Therefore, it is preferable to prepare and cryopreserve a large number of cardiomyocytes separated into single cells in advance. Accordingly, in the method of the present invention, cryopreserved cardiomyocytes may be thawed just before the cells are used in the method of the present invention, and then may be used.

When a cell survival rate at the time of seeding cardiomyocytes in a container is 50 % or more, a proportion of dead cells present in a suspension (liquid in which the cardiomyocytes are cultured) is low, and therefore cardiomyocyte spheroids with high quality can be produced. Therefore, the cell survival rate at the time of seeding the cardiomyocytes used in the present invention in a container is preferably 50% or more, more preferably 55% or more, still more preferably 60% or more, particularly preferably 65% or more, and most preferably 70% or more.

When 70% or more of the cells in the suspension at the time of starting the culture are single cells, sizes of the cardiomyocyte spheroids obtained by the present invention will be uniform. Therefore, in the present invention, a percentage of single cells in the suspension at the time of starting the culture is preferably 70% or more, more preferably 80% or more, particularly preferably 85% or more, still more preferably 90% or more, and most preferably 95% or more. A spheroid of two, three, four or more cardiomyocytes as well as single cells may be contained in the suspension at the time of starting the culture.

### (Culture Container)

A culture container used in the present invention will now be described. The cardiomyocyte spheroid of the present invention can be produced in a container equipped (provided) with a stirring blade or the like. As used herein, the term "a container equipped with a stirring blade or the like" encompasses a container united with a stirring blade, and a container to which a stirring bar is fixed by magnetic force or the like. Shapes of the container and the stirring blade or the like, without limitation, preferably enable cells to be homogeneously dispersed. For example, a shape of the container can be cylindrical. The stirring blade can have, without limitation, for example, a turbine shape, a paddle shape, a propeller shape, or the like. The stirring bar can have, without limitation, for example, a bar shape, a cross shape, a propeller shape, or the like. Equipment position of the stirring blade or the like can be, without limitation, for example, the bottom face or upper portion (lid or the like) of the container. Examples of the container equipped with the stirring blade that can be used in the present invention include a container having a bottom face equipped with a paddle-shaped stirring blade. Here, the paddle-shaped stirring blade may be, for example, a stirring blade having a rotation shaft equipped with a flat plate (having, for example, a shape such as a rectangular, square, or triangular shape, or a shape spreading out toward a bottom face). Specific examples of the container equipped with the stirring blade that can be used in the present invention include a Single use reactor for culture of stem cells from ABLE, a disposable spinner flask from Corning, and BioBLU Single Use Vessel from Eppendorf.

When a medium used is poor in protein ingredients, cardiomyocytes may adhere to an inner face of a culture container due to their own characteristics even in the case of adding no adhesive substrate, and therefore, an inner face of the container used in the present invention is preferably cell non-adherent. A method of treating the inner face of a container to make it cell non-adherent includes, without limitation, a method in which the inner face of a container is coated with a cell non-adherent substance. Examples of such method include a method in which a hydrophilic polymer such as polyhydroxyethyl methacrylate (p-HEMA) is dissolved in ethanol, added into a container, and dried to coat the inner face of the container. In addition to p-HEMA, a phospholipid polymer such as Lipidure from NOF CORPORATION can also be used. A culture container manufactured using a cell non-adherent material may also be used. Examples of such cell non-adherent materials include, but are not limited to, polystyrene.

A volume of the container used in the present invention can be, for example, 3 mL or more, and is preferably 5 mL or more. A large number of cardiomyocyte spheroids can be produced in a case where the volume of the container is 5 mL or more. Therefore, the volume of the container used in the present invention is preferably 5 mL or more, more preferably 15 mL or more, still more preferably 50 mL or more, particularly preferably 100 mL or more in view of commercial production, preferably 500 mL or more in the case of, for example, using the cardiomyocyte spheroid for treating a relatively small patient, and preferably 1000 mL or more in the case of, for example, using the cardiomyocyte spheroid for treating a relatively large patient. Cell spheroids can be produced with favorable handleability in a case where the volume of the container is 70 L or less. Therefore, the volume of the container used in the present invention is preferably 70 L or less, more preferably 30 L or less, still more preferably 15 L or less, particularly preferably 10 L or less, and most preferably 7 L or less.

The container used in the present invention is preferably sterilized, and even more preferably disposable. The container used in the present invention is also preferably a container that can secure sterility during production of a cardiomyocyte spheroid (during culture).

### (Liquid for Cell Suspension)

In the present invention, a liquid for cell suspension used in the step of culturing cardiomyocytes separated into single cells while stirring the liquid in which the cardiomyocytes are suspended, thereby causing aggregation of the cells, is, without limitation, preferably a medium in which cardiomyocytes can be stably cultured, and examples of the liquid include carmyA (Myoridge Co. Ltd.), RPMI medium + B27 (Thermo Fisher Scientific), and MEM-α medium + 5% fetal bovine serum (Fetal Bovine Serum). The above-described liquid for cell suspension can be usually replaced in an operation called medium replacement in the art. Without limitation, the medium replacement may be regularly performed, for example, every 1 hour, every 4 hours, every 8 hours, every 12 hours, every 24 hours, every 48 hours, or the like, or may be irregularly performed. An amount of liquid replaced is not limited, but may be the substantially total amount of the liquid, or may be one-half of the total amount of the liquid. The medium replacement may be performed by replacing a predetermined amount of the medium constantly by perfusion.

A ROCK (Rho-associated kinase) inhibitor has an effect of keeping a viability of cardiomyocytes in a single-cell state at a high level and promoting aggregation thereof, and therefore, it is preferable to add the ROCK inhibitor to a medium. A concentration of the ROCK inhibitor in the medium may be in a range in which the above-described effect is exhibited and no cytotoxicity is exhibited. Examples of the lower limit value of the concentration of the ROCK inhibitor in the medium include, but are not limited to, 1 µM, 2 µM, or 3 µM, and examples of the upper limit value of the concentration of the ROCK inhibitor in the medium include, but are not limited to, 50 µM, 25 µM, or 10 µM. It is possible to continue adding the ROCK inhibitor during the culture, and it is also possible to remove it during the culture. Examples of the ROCK inhibitor include, but are not limited to, Y-27632 (N-(4-pyridinyl)-4β-[(R)-1-aminoethyl]cyclohexane-1α-carboamide).

In the present invention, an amount of the suspension in the container is preferably a volume recommended by the manufacturer of the container in consideration of sparging of the suspension during flow of the suspension. In the present invention, the amount of the suspension in the container can be, for example, 3 mL to 70 L, 3 mL to 30 L, 3 mL to 15 L, 3 mL to 10 L, 3 mL to 7.5 L, 3 mL to 3 L, 3 mL to 1.5 L, 3 mL to 500 mL, 3 mL to 100 mL, 3 mL to 50 mL, 3 mL to 30 mL, 3 mL to 10 mL, 3 mL to 7 mL, 5 mL to 70 L, 5 mL to 30 L, 5 mL to 15 L 5 mL to L, 5 mL to 7.5 L, 5 mL to 3 L, 5 mL to 1.5 L, 5 mL to 500 mL, 5 mL to 100 mL, 5 mL to 50 mL, 5 mL to 30 mL, 5 mL to 10 mL, 5 mL to 7 mL, or 5 mL.

### (Method of Seeding Cardiomyocytes)

A method of seeding the cardiomyocytes in the present invention at the time of the start of the culture of the cardiomyocytes will now be described. It is preferable to seed the cardiomyocytes separated into single cells in the present invention. Examples of the seeding method include, but are not limited to: a method in which a predetermined amount of medium is added into a container in advance, and cardiomyocytes suspended in a liquid are then added thereto; a method in which a suspension obtained by suspending cardiomyocytes in a liquid so as to achieve a predetermined cell density is added into a container; and a method in which a predetermined number of cardiomyocytes are added into a container, followed by adding a liquid up to the predetermined amount of the liquid.

The method of the present invention is characterized in that cardiomyocytes, and/or a cardiomyocyte spheroid being formed are in a state of being suspended in a liquid. As used herein, the term "a state of being suspended in a liquid " of a certain substance (cell or cell spheroid) means a state in which the substance is suspended in a liquid such as a medium. Examples of the state include a state in which the substance settles on the bottom of a container and low stimulation enables the substance to leave an inner face of the container. In a case where a concentration of the cardiomyocytes in the suspension is 1.0 × 10³ cells/mL or more, cost of liquid components such as a medium is low, and the cells easily form a spheroid. Therefore, the concentration of the cardiomyocytes in the suspension in the method of the present invention is, without limitation, preferably 1.0 × 10³ cells/mL or more, more preferably 1.0 × 10⁴ cells/mL or more, particularly preferably 1.0 × 10⁵ cells/mL or more, and most preferably 1.5 × 10⁵ cells/mL or more. In a case where the cell concentration of cardiomyocytes in the suspension is 1.0 × 10⁸ cells/mL or less, suppression of over-aggregation of the cardiomyocytes is facilitated. Therefore, the cell concentration of the cardiomyocytes in the suspension in the method of the present invention is, without limitation, preferably 1.0 × 10⁸ cells/mL or less, still more preferably 1.0 × 10⁷ cells/mL or less, particularly preferably 5.0 × 10⁶ cells/mL or less, and most preferably 1.0 × 10⁶ cells/mL or less. The cell concentration of the cardiomyocytes in the suspension in the method of the present invention can be, for example, 1.0 × 10³ cells/mL to 1.0 × 10⁸ cells/mL, 1.0 × 10³ cells/mL to 1.0 × 10⁷ cells/mL, 1.0 × 10³ cells/mL to 1.0 × 10⁶ cells/mL, 1.0 × 10³ cells/mL to 5.0 × 10⁵ cells/mL, 1.0 × 10⁴ cells/mL to 1.0 × 10⁸ cells/mL, 1.0 × 10⁴ cells/mL to 1.0 × 10⁷ cells/mL, 1.0 × 10⁴ cells/mL to 1.0 × 10⁶ cells/mL, 1.0 × 10⁴ cells/mL to 5.0 × 10⁵ cells/mL, 1.0 × 10⁵ cells/mL to 1.0 × 10⁸ cells/mL, 1.0 × 10⁵ cells/mL to 1.0 × 10⁷ cells/mL, 1.0 × 10⁵ cells/mL to 1.0 × 10⁶ cells/mL, 1.0 × 10⁵ cells/mL to 5.0 × 10⁵ cells/mL, 5.0 × 10⁵ cells/mL to 1.0 × 10⁸ cells/mL, 5.0 × 10⁵ cells/mL to 1.0 × 10⁷ cells/mL, and 5.0 × 10⁵ cells/mL to 1.0 × 10⁶ cells/mL.

For example, in a case where cryopreserved cardiomyocytes are thawed and then used in the method of the present invention, the thawed cardiomyocytes may contain debris which is a conjugate of dead cells, depending on a method of freezing the cells, a method of preserving the cells, and a method of thawing the cells. In such a case, it is preferable to use the cardiomyocytes after a suspension containing the thawed cardiomyocytes is passed through a cell strainer to remove the debris. Accordingly, in the method of the present invention, prior to start of the culture of cardiomyocytes, debris derived from the cardiomyocytes can be removed in advance using a cell strainer. An opening of the cell strainer may have a size enabling the debris to be removed sufficiently. The opening of the cell strainer is not limited, but a single cell can desirably pass through the opening of the cell strainer. For example, it is preferable to use any one or more of the cell strainers having an opening of 30 µm, 35 µm, 70 µm, or 75 µm.

### (Conditions of Culture of Cardiomyocytes)

In the present invention, a culture temperature in the step of culturing the cardiomyocytes separated into single cells while stirring the liquid in which the cardiomyocytes are suspended, thereby causing aggregation of the cells, is, without limitation, preferably 30 to 45°C, more preferably 36 to 38°C, and most preferably 37°C.

Compositions and concentrations of gases in an environment during the above-described culture are not particularly limited. The above-described culture can also be performed in, for example, an incubator under a carbonic acid gas atmosphere that is commonly used. A concentration of carbonic acid gas during the above-described culture is, without limitation, for example, 0 to 10%, for example, 4 to 6%, and for example, about 5%. The concentration of carbonic acid gas can also be variably controlled in a range of, without limitation, for example, 0 to 10%, using an instrument that can automatically control the concentration of carbonic acid gas. In a case where a concentration of oxygen during the above-described culture is 1% or more, cell death hardly occurs. Therefore, the concentration of oxygen during the above-described culture is, without limitation, preferably 1% or more, more preferably 10% or more, still more preferably 15% or more, particularly preferably 18% or more, and most preferably 20% or more. Moreover, in a case where the concentration of oxygen during the above-described culture is 40% or less, unnecessary oxidation of cells can be suppressed. Therefore, the concentration of oxygen during the above-described culture is preferably 40% or less, more preferably 30% or less, still more preferably 25% or less, particularly preferably 22% or less, and most preferably 21% or less.

In the present invention, an amount of dissolved oxygen in the cardiomyocytes suspension in the step of culturing the cardiomyocytes separated into single cells while suspending the cardiomyocytes in the liquid, thereby causing aggregation of the cells, is, without limitation, preferably 10 or more and 100 or less, more preferably 25 or more and 100 or less, still more preferably 40 or more and 100 or less, particularly preferably 50 or more and 100 or less, and most preferably 80 or more and 100 or less, when the amount of dissolved oxygen in the case of saturating the liquid components of the suspension with a sufficient amount of air is set at 100. This is because, suppression of cell death is facilitated when the amount of dissolved oxygen is 10 or more, and unnecessary aeration is not needed when the amount of dissolved oxygen is 100 or less.

In the present invention, culture time for the cardiomyocytes can be, without limitation, for example, 20 hours to 96 hours, 20 to 72 hours, 20 to 60 hours, 20 to 48 hours, 24 hours to 96 hours, 24 to 72 hours, 24 to 60 hours, or 24 to 48 hours.

### (Blade Tip Speed during Stirred Culture)

A blade tip speed during stirred culture will now be described. "A blade tip speed" of a stirring blade or the like means the circumferential speed of the site of the stirring blade or the like situated farthest from a rotation shaft or a rotation center (blade tip).

The method of producing a cardiomyocyte spheroid of the present invention is characterized by being performed while rotating the stirring blade or the like in the container to allow the suspension comprising the cells to flow. For example, the container containing the suspension comprising the cardiomyocytes is placed on a magnetic stirrer, and stirring is continued with a stirring blade or the like having a lower end to which a magnetic force body (a permanent magnet or the like) is fixed, whereby the cells are aggregated before long, and cell spheroids are formed. The blade tip speed of the stirring blade or the like can be adjusted according to a cell strain or medium used. Since cells will flow in a case where the blade tip speed is 7.9 × 10⁻³ m/s or more, the blade tip speed is, without limitation, preferably 7.9 × 10⁻³ m/s or more, more preferably 1.6 × 10⁻² m/s or more, still more preferably 2.4 × 10⁻² m/s or more, particularly preferably 3.2 × 10⁻² m/s or more, and most preferably 3.9 × 10⁻² m/s or more. Moreover, the blade tip speed of the stirring blade or the like is, without limitation, preferably 1.9 × 10⁻¹ m/s or less, more preferably 1.7 × 10⁻¹ m/s or less, still more preferably 1.3 × 10⁻¹ m/s or less, particularly preferably 9.5 × 10⁻² m/s or less, and most preferably 8.0 × 10⁻² m/s or less, in view of the stability of the cardiomyocyte spheroid. In the present invention, a rotation speed of the stirring blade or the like can be, without limitation, for example, 1 rpm to 100 rpm, 1 rpm to 80 rpm, 1 rpm to 60 rpm, 1 rpm to 55 rpm, 5 rpm to 100 rpm, 5 rpm to 80 rpm, 5 rpm to 60 rpm, 5 rpm to 55 rpm, 15 rpm to 100 rpm, 15 rpm to 80 rpm, 15 rpm to 60 rpm, 15 rpm to 55 rpm, 25 rpm to 100 rpm, 25 rpm to 80 rpm, 25 rpm to 60 rpm, or 25 rpm to 55 rpm.

In the stirred culture using the stirring blade or the like described above, an increase in the blade tip speed results in an improvement in a flowability of the cells and an increase in frequency of contact between the cells to promote adhesion between the cells. However, an increase in the blade tip speed also results in an increase in shearing force. Therefore, an excessive increase in the blade tip speed conversely results in inhibition of adhesion between the cells. Moreover, an increase in the blade tip speed results in suppression of residence of formed cardiomyocyte spheroids on the bottom face of the container, whereby aggregation of the cardiomyocyte spheroids is suppressed. As a result of intensive examination, the present inventors found a method of adjusting the blade tip speed, by which a size of a cardiomyocyte spheroid can be adjusted.

In the present invention, for example, a blade tip speed at which an aggregation is promoted, and a blade tip speed at which a size of a cardiomyocyte spheroid is maintained can be appropriately selected according to a target size of the cardiomyocyte spheroid. Moreover, in the present invention, a blade tip speed (speed of stirring) can be changed during culture, and, as a result, a size of the cardiomyocyte spheroid can be more precisely controlled. Without limitation, the blade tip speed (speed of stirring) can be changed (for example, increased), for example, in a stepwise or continuous manner during the culture. Without limitation, the blade tip speed can be changed, for example, at timing at which a precursor having a spheroid diameter of 500 µm or more is formed, timing at which a cardiomyocyte spheroid having a size close to a target size is formed, or the like. The term "precursor" as used herein is a precursor that is a cellular spheroid at a stage prior to a cardiomyocyte spheroid, and condenses later to become the cardiomyocyte spheroid.

In the present invention, the initial speed of the blade tip speed (speed at the time of the start of the culture) can be, without limitation, for example, 7.9 × 10⁻³ m/s to 6.0 × 10⁻² m/s, 1.6 × 10⁻² m/s to 6.0 × 10⁻² m/s, 3.2 × 10⁻² m/s to 6.0 × 10⁻² m/s, 3.9 × 10⁻² m/s to 6.0 × 10⁻² m/s, 4.5 × 10⁻² m/s to 6.0 × 10⁻² m/s, 5.5 × 10⁻² m/s to 6.0 × 10⁻² m/s, 7.9 × 10⁻³ m/s to 5.5 × 10⁻² m/s, 1.6 × 10⁻² m/s to 5.5 × 10⁻² m/s, 3.2 × 10⁻² m/s to 5.5 × 10⁻² m/s, 3.9 × 10⁻² m/s to 5.5 × 10⁻² m/s, 7.9 × 10⁻³ m/s to 4.5 × 10⁻² m/s, 1.6 × 10⁻² m/s to 4.5 × 10⁻² m/s, 3.2 × 10⁻² m/s to 4.5 × 10⁻² m/s, or 3.9 × 10⁻² m/s to 4.5 × 10⁻² m/s.

In the present invention, the final speed of the blade tip speed (speed at the time of end of culture) can be, without limitation, for example, 6.5 × 10⁻² m/s to 1.9 × 10⁻¹ m/s, 7.0 × 10⁻² m/s to 1.9 × 10⁻¹ m/s, 7.5 × 10⁻² m/s to 1.9 × 10⁻¹ m/s, 8.0 × 10⁻² m/s to 1.9 × 10⁻¹ m/s, 8.5 × 10^{- 2} m/s to 1.9 × 10⁻¹ m/s, 6.5 × 10⁻² m/s to 9.5 × 10⁻² m/s, 7.0 × 10⁻² m/s to 9.5 × 10⁻² m/s, 7.5 × 10⁻² m/s to 9.5 × 10⁻² m/s, 8.0 × 10⁻² m/s to 9.5 × 10⁻² m/s, 8.5 × 10⁻² m/s to 9.5 × 10⁻² m/s, 6.5 × 10⁻² m/s to 8.5 × 10⁻² m/s, 7.0 × 10⁻² m/s to 8.5 × 10⁻² m/s, 7.5 × 10⁻² m/s to 8.5 × 10⁻² m/s, 8.0 × 10⁻² m/s to 8.5 × 10⁻² m/s, 6.5 × 10⁻² m/s to 7.5 × 10⁻² m/s, or 7.0 × 10⁻² m/s to 7.5 × 10⁻² m/s.

In one embodiment, the blade tip speed can be increased only once, 1 to 48 hours, 1 to 36 hours, 1 to 24 hours, 1 to 16 hours, 1 to 14 hours, 1 to 12 hours, 1 to 10 hours, 1 to 8 hours, 1 to 6 hours, 3 to 48 hours, 3 to 36 hours, 3 to 24 hours, 3 to 16 hours, 3 to 14 hours, 3 to 12 hours, 3 to 10 hours, 3 to 8 hours, 3 to 6 hours, 6 to 48 hours, 6 to 36 hours, 6 to 24 hours, 6 to 16 hours, 6 to 14 hours, 6 to 12 hours, 6 to 10 hours, 6 to 8 hours, 6 hours, 14 hours, or 24 hours after the start of the culture.

In one embodiment, the blade tip speed can be increased plural times (for example, two, three, four, or five times, or the like) during the culture.

### (Step of Collecting Cardiomyocyte spheroid)

A method of collecting the cardiomyocyte spheroid obtained by the present invention will now be described. Examples of a method of collecting a suspension comprising the cardiomyocyte spheroid include, but are not limited to, a method in which the suspension is sufficiently homogenized, or the cardiomyocyte spheroid is settled on the bottom face, followed by collecting the suspension using a pipetter, a syringe, or the like. An amount of the suspension collected may be the whole amount of the suspension in the container or part thereof. In the case of collecting the whole amount of the suspension in the container, a cardiomyocyte spheroid remaining in the container after the operation described above can be collected by washing the container with a medium. A medium component may be removed from the collected cardiomyocyte spheroid by a centrifugal operation or the like, and may be replaced with a solution or the like that is used in transplantation.

### (Method of Removing Single Cells That Have Not Formed Cardiomyocyte spheroid)

Single cells remaining after formation of the cardiomyocyte spheroid are preferably removed because a survival rate of the single cells is low. Examples of a method of removing single cells include, but are not limited to: a method in which the cardiomyocyte spheroid is settled in the culture container in which the stirring blade or the like is stopped, and only floating single cells are removed; a method in which the whole amount of the suspension is passed through a cell strainer, through which only single cells can pass, to remove single cells; and a method in which the whole amount of the suspension is collected in a centrifugal tube, followed by separating the cardiomyocyte spheroid and single cells from each other by a centrifugal operation, to remove the single cells.

### (Quality of Cardiomyocyte spheroid)

The quality of the cardiomyocyte spheroid(s) produced by the method of producing a cardiomyocyte spheroid of the present invention (referred to as "the cardiomyocyte spheroid of the present invention") will now be described.

According to the production method of the present invention, a beating cardiomyocyte spheroid can be obtained. Accordingly, the cardiomyocyte spheroid of the present invention may be beating. Generally, a cardiomyocyte spheroid, the whole of which is uniformly beating, is preferably used in transplantation therapy. Therefore, the cardiomyocyte spheroid of the present invention can be preferably a cardiomyocyte spheroid, the whole of which is uniformly beating. The term "beating" described herein means that contraction and dilatation of a cardiomyocyte spheroid are repeated at regular intervals or at substantially regular intervals.

According to the production method of the present invention, a cardiomyocyte spheroid having a favorable shape and high true sphericity can be obtained. Examples of an index of a shape may include an aspect ratio. The aspect ratio can be determined as a ratio between a minimum Feret diameter and a maximum Feret diameter. An average value of the aspect ratio(s) of the cardiomyocyte spheroid(s) of the present invention provided by the production method of the present invention can be preferably 0.60 or more, more preferably 0.65 or more, still more preferably 0.70 or more, particularly preferably 0.75 or more, and most preferably 0.80 or more.

According to the production method of the present invention, a population of cardiomyocyte spheroids having relatively uniform sizes can be provided. Examples of an index of a uniformity of sizes include a standard deviation. For the cardiomyocyte spheroid(s) of the present invention provided by the production method of the present invention, a standard deviation of a size(s) of the cell spheroid (s) is preferably 50% or less of an average value of a size(s) of the cell spheroid (s), still more preferably 40% or less, particularly preferably 30% or less, and most preferably 20% or less. As used herein, the term "cardiomyocyte spheroid" is a cell spheroid having a size of 30 µm or more, in which two or more cardiomyocytes adhere to each other. An average value of a size(s) of the cardiomyocyte spheroid(s) of the present invention is, without limitation, preferably 30 µm or more, more preferably 75 µm or more, still more preferably 100 µm or more, particularly preferably 125 µm or more, and most preferably 150 µm or more, in view of expression of a function (beating or the like) as cardiomyocytes. In a case where the average value of the size(s) of the cardiomyocyte spheroid(s) of the present invention is 2000 µm or less, it is easy to inject the cardiomyocyte spheroid(s) in transplantation. Therefore, the average value of the size(s) of the cardiomyocyte spheroid(s) of the present invention is preferably 2000 µm or less, more preferably 1500 µm or less, still more preferably 1000 µm or less, particularly preferably 500 µm or less, and most preferably 250 µm or less. As used herein, the term "a size of a cell spheroid" is an average spheroid diameter on a volumetric basis, and is determined as an average of a minimum Feret diameter and a maximum Feret diameter.

In the case of using the production method of the present invention, seeded viable cells form (a) cardiomyocyte spheroid(s) at high efficiency. In the present invention, a total number of cardiomyocytes forming the cardiomyocyte spheroid(s) is preferably 15% or more, more preferably 30% or more, still more preferably 45% or more, particularly preferably 60% or more, and most preferably 75% or more of a number of viable cells seeded in the container (at the time of the start of the culture), in view of production efficiency of the cardiomyocyte spheroid(s).

According to the production method of the present invention, a high-quality cardiomyocyte spheroid(s) with a small number of dead cells can be provided. Examples of a method of evaluating viable cells and dead cells in a cardiomyocyte spheroid include, but are not limited to, a method using Live/Dead Cell Staining Kit II (PromoKine). In the method, viable cells and dead cells are stained with Calcein-AM and EthD-III, respectively. A cell viability in the cardiomyocyte spheroid may be evaluated by, for example, double-staining the cardiomyocyte spheroid with Calcein-AM and EthD-III and calculating a percentage of an area of a region stained with EthD-III to an area of a region stained with Calcein-AM from a staining image obtained by fluorescence imaging with a phase-contrast microscope or the like. In a case where the percentage is 30% or less, it can be said that the cardiomyocyte spheroid has high quality. Accordingly, in a case where the cardiomyocyte spheroid of the present invention is double-stained with Calcein-AM and EthD-III, an area of a region stained with EthD-III is preferably 30% or less, more preferably 25% or less, still more preferably 20% or less, particularly preferably 15% or less, and most preferably 10% or less of an area of a region stained with Calcein-AM.

Examples of another method of evaluating viable cells and dead cells in a cardiomyocyte spheroid include a method in which a frozen section of a cardiomyocyte spheroid is produced and immunostained. The frozen section used in the staining is preferably a frozen section cut from a plane passing through a center of gravity of a cardiomyocyte spheroid, because such frozen section most accurately represents a state of the interior of the cell spheroid. Examples of a method of staining only dead cells in a frozen section include a method in which only dead cells are stained with an apoptotic marker. Examples of the apoptotic marker include Cleaved Caspase-3 and Cleaved Caspase-7. For example, a cardiomyocyte spheroid may be cut so that a cross section passes through a center of gravity of a cardiomyocyte spheroid, and a cell viability in the cardiomyocyte spheroid may be evaluated based on a percentage of a number of cells stained with an apoptotic marker in the cross section to a total number of cells in the cross section. In a case where the percentage is 50% or less, it can be said that the percentage of viable cells is high, and the cardiomyocyte spheroid has high quality. Therefore, the percentage is preferably 50% or less, more preferably 40% or less, still more preferably 30% or less, particularly preferably 30% or less, most preferably 10% or less.

### (Method of Using Obtained Cardiomyocyte spheroid)

The cardiomyocyte spheroid of the present invention can be transplanted to the heart tissue of an individual (living body) by various techniques. For example, the cardiomyocyte spheroid of the present invention can be administered into the myocardial layer of the heart using an injection or a catheter. In a case where a cardiomyocyte spheroid is produced by the method of the present invention without using any special liquid component, the cardiomyocyte spheroid can also be used for transplantation directly without substituting the liquid component of the suspension, or can also be transplanted after substitution of the liquid component of the suspension with a liquid suitable for an individual (for example, physiological saline). The cardiomyocyte spheroid(s) of the present invention has a small distribution of a size, and also has a favorable shape. Therefore, a trouble such as clogging of an injection needle with the cardiomyocyte spheroid hardly occurs. Just to make sure, however, it is preferable to collect and use only cardiomyocyte spheroids that have been able to pass through an injection needle, a flow path, a sieve, or the like having an inner diameter or opening that is not more than the inner diameter of an injection needle used in transplantation. Particularly in the case of using a low-invasive injection needle with 30 G or less, it is effective to remove cardiomyocyte spheroids that do not pass through the injection needle.

Any method capable of easily obtaining a large number of preferred cardiomyocyte spheroids at a time, like the present invention, has not been known until now. It can be said that the present invention is a very excellent epoch-making invention.

### Examples

Hereinafter, the present invention will be described with reference to Examples. However, the present invention is not intended to be limited by these Examples.

Ingredients and materials used are listed in Table 1.

**[Table 1]**

| Classification | Product Name | Manufacturer | Model Number |
|---|---|---|---|
| Seeding medium | Seeding Medium for CarmyA | Myoridge | ME-02 |
| Additive | Y-27632 | Wako | 036-24023 |
| Culture container | 12-well plate | Sumitomo Bakelite Co., Ltd. | MS8012R |
| | Cell culture plate 96-well, Round Bottom | WATSON | 197-96UCS |
| | 5 mL Single use reactor for culture of stem cells | ABLE | 3524 |
| Coating agent | p-HEMA (Polyhydroxyethylmethacrylate) | SIGMA | P3932 |
| Solvent for coating agent | 95% Ethanol | Wako | 051-00476 |
| Buffer | PBS | gibco | 10010-031 |

Y-27632 in Table 1 is a ROCK inhibitor.

p-HEMA was dissolved in ethanol, and added into a 5 mL Single use reactor for culture of stem cells. The single use reactor was dried, thereby coating it with p-HEMA.

### (Comparative Example 1)

Thawing of cells and seeding of the cells in a culture container were performed in the following procedures.
Procedure 1) Y-27632 was added to Seeding Medium for CarmyA so as to achieve a final concentration of 10 µM to prepare a thawing medium. The thawing medium was warmed to 37°C.
Procedure 2) One vial containing cryopreserved cardiomyocytes derived from human iPS cells (CarmyA manufactured by Myoridge Co. Ltd., purity of >90%, hereinafter the same applies) was warmed for 120 seconds in a water bath at 37°C.
Procedure 3) Without pipetting, 50 mL of cell suspension was transferred from the vial warmed in Procedure 2) to each centrifuge tube.
Procedure 4) The vial in Procedure 3) was washed with 1 mL of the thawing medium in Procedure 1), and the 1 mL of the medium used for the washing was added to the cell suspension in Procedure 3) at a rate of 1 drop/5 seconds, and mixed by shaking.
Procedure 5) To the cell suspension in Procedure 4), 1 mL of the thawing medium in Procedure 1) was added at 1 drop/2 seconds, and mixed by shaking.
Procedure 6) To the cell suspension in Procedure 5), 8 mL of the thawing medium in Procedure 1) was added at 1 drop/second, and mixed by shaking.
Procedure 7) Centrifugation of the cell suspension in Procedure 6) was performed at 300 × g for 3 minutes, followed by removing a supernatant.
Procedure 8) The cells precipitated in Procedure 7) were resuspended in 500 µL of the thawing medium, and cell counting was performed using a Countess II FL automatic cell counter manufactured by Thermo Fisher Scientific K.K.
Procedure 9) The cell suspension in Procedure 8) in an amount comprising 1.0 × 10³ viable cells was seeded in one well of a round-bottom 96-well plate (cell culture plate 96-well, round bottom), on the basis of the results of the cell counting, the thawing medium was added thereto to achieve a total amount of 100 µL, and then stationary culture was performed.

### The culture was performed under 5.0% CO₂ at 37°C. (Hereinafter, the same applies to all Comparative Examples and Examples.)

### (Comparative Example 2)

The procedures up to Procedure 8) described in Comparative Example 1 were performed in a similar manner, and subsequent procedures were performed as follows.
Procedure 9) The cell suspension in Procedure 8) in an amount comprising 2.0 × 10⁵ cells was seeded in one well of a 12-well plate, on the basis of the results of the cell counting, and the thawing medium was added thereto to achieve a total amount of 1 mL.
Procedure 10) The 12-well plate in Procedure 9) was put on Orbital Shaker OS-762 (that rotates in a circular motion along a horizontal plane at a rotating width (diameter) of 25 mm) manufactured by Optima Inc., and culture was started at a rotating speed of 100 rpm. (This time point is set as "0 hour after the start of the culture".)

### (Comparative Example 3)

Thawing of cells and seeding of the cells in a culture container were performed in the following procedures.
Procedure 1) Y-27632 was added to Seeding Medium for CarmyA so as to achieve a final concentration of 10 µM to prepare a thawing medium. The thawing medium was warmed to 37°C.
Procedure 2) One vial comprising cryopreserved cardiomyocytes derived from human iPS cells was warmed for 120 seconds in a water bath at 37°C.
Procedure 3) The cell suspension was transferred from the vial warmed in Procedure 2) into a 15-mL tube containing10 mL of the thawing medium prepared in Procedure 1). At that time, an operation of homogenizing the cell suspension using a pipetter was not performed.
Procedure 4) The vial in Procedure 3) was washed with 1 mL of the thawing medium in the tube in Procedure 3), and 1 mL of the medium used for the washing was put back in the tube described above.
Procedure 5) Centrifugation of the tube in Procedure 4) was performed at 300 × g for 3 minutes, followed by removing a supernatant.
Procedure 6) The cells precipitated in Procedure 5) were resuspended in 500 µL of the thawing medium, and cell counting was performed using a Countess II FL automatic cell counter manufactured by Thermo Fisher Scientific K.K.
Procedure 7) The cell suspension in Procedure 6) in an amount comprising 1.5 × 10⁵ viable cells was passed through a strainer having an opening of 35 µm, and seeded in one well of a 12-well plate, on the basis of the results of the cell counting, and the thawing medium was added thereto to achieve a total amount of 1 mL.
Procedure 8) The cell suspension in Procedure 6) in an amount comprising 2.0 × 10⁵ viable cells was seeded in one well of the 12-well plate, on the basis of the results of the cell counting, and the thawing medium was added thereto to achieve a total amount of 1 mL.
Procedure 9) The plate was put on Orbital Shaker OS-762 (that rotates in a circular motion along a horizontal plane at a rotating width (diameter) of 25 mm) manufactured by Optima Inc., and culture was started at a rotating speed of 100 rpm. (This time point is set as "0 hours after the start of the culture".)

### (Example 1)

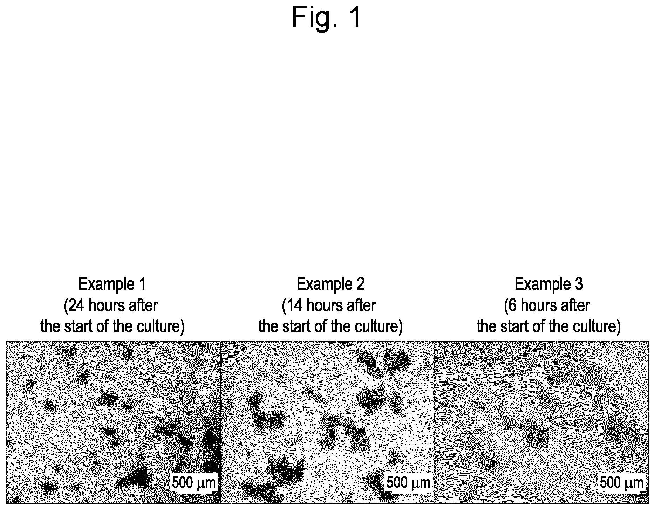

Thawing of cells and seeding of the cells in a 5 mL Single use reactor for culture of stem cells (hereinafter, referred to as "reactor") were performed in the following procedures.
Procedure 1) Y-27632 was added to Seeding Medium for CarmyA so as to achieve a final concentration of 10 µM to prepare a thawing medium. The thawing medium was warmed to 37°C.
Procedure 2) One vial containing cryopreserved cardiomyocytes derived from human iPS cells was warmed for 120 seconds in a water bath at 37°C.
Procedure 3) The cell suspension was transferred from the vial warmed in Procedure 2) into a 15-mL tube containing 10 mL of the thawing medium prepared in Procedure 1). At that time, an operation of homogenizing the cell suspension using a pipetter was not performed.
Procedure 4) The vial in Procedure 3) was washed with 1 mL of the thawing medium in the tube in Procedure 3), and 1 mL of the medium used for the washing was put back in the tube described above.
Procedure 5) Centrifugation of the tube in Procedure 4) was performed at 300 × g for 3 minutes, followed by removing a supernatant.
Procedure 6) The cells precipitated in Procedure 5) were resuspended in 500 µL of the thawing medium, and cell counting was performed using a Countess II FL automatic cell counter manufactured by Thermo Fisher Scientific K.K.
Procedure 7) The cell suspension in Procedure 6) in an amount comprising 7.5 × 10⁵ viable cells was passed through a strainer having an opening of 70 µm, on the basis of the results of the cell counting, the thawing medium was added thereto to achieve a total amount of 5 mL, and the resultant was seeded in the reactor.
Procedure 8) Culture was started at a blade tip speed of 3.9 × 10⁻² m/s (reactor stirring speed of 25 rpm). (This time point is set as "0 hours after the start of the culture".)
Procedure 9) 24 hours after the start of the culture, the presence of cardiomyocyte spheroids having sizes close to a target size was observed as seen in Figure 1, and the blade tip speed was increased to 8.0 × 10⁻² m/s (reactor stirring speed of 51 rpm). Then, the stirred culture was continued until 48 hours after the start of the culture.

### (Example 2)

The following experiment was carried out for the purpose of obtaining cardiomyocyte spheroids smaller than those in Example 1 by changing timing of increasing a blade tip speed. Procedures 1) to 8) as described in Example 1 were performed. 14 hours after the start of the culture, the presence of precursors having spheroid diameters of 500 µm or more was observed as seen in Figure 1, and the blade tip speed was increased to 8.0 × 10⁻² m/s (reactor stirring speed of 51 rpm). Then the stirred culture was continued until 48 hours after the start of the culture.

### (Example 3)

The following experiment was carried out for the purpose of obtaining cardiomyocyte spheroids smaller than those in Example 2 by changing timing of increasing a blade tip speed. Procedures 1) to 8) as described in Example 1 were performed. 6 hours after the start of culture, the presence of precursors having spheroid diameters of 500 µm or more was observed as seen in Figure 1, and the blade tip speed was increased to 8.0 × 10⁻² m/s (reactor stirring speed of 51 rpm). Then, the stirred culture was continued until 48 hours after the start of the culture.

### (Example 4)

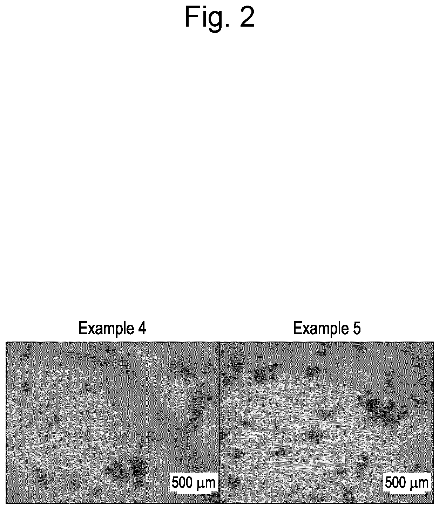

The following experiment was carried out for the purpose of obtaining cardiomyocyte spheroids that were smaller than those in Example 2 and had low variations in spheroid diameter by increasing a blade tip speed in a stepwise manner. Procedures 1) to 8) as described in Example 1 were performed. The blade tip speed was increased to 4.7 × 10⁻² m/s (reactor stirring speed of 30 rpm) 1 hour after the start of culture, and the blade tip speed was increased to 5.5 × 10⁻² m/s (reactor stirring speed of 35 rpm) 2 hours after the start of the culture. 3 hours after the start of the culture, the presence of precursors having spheroid diameters of 500 µm or more was observed as seen in Figure 2, and the blade tip speed was increased to 6.3 × 10⁻² m/s (reactor stirring speed of 40 rpm). The blade tip speed was increased to 7.1 × 10⁻² m/s (reactor stirring speed of 45 rpm) 6 hours after the start of the culture, and the stirred culture was continued until 48 hours after the start of the culture.

### (Example 5)

For the purpose of improving a productivity of cardiomyocyte spheroids by changing a seeding density of cardiomyocytes derived from human iPS cells in a suspension, thawing of cells and seeding of the cells in a 5 mL Single use reactor for culture of stem cells (hereinafter, referred to as "reactor") were performed in the following procedures.
Procedure 1) Y-27632 was added to Seeding Medium for CarmyA so as to achieve a final concentration of 10 µM to prepare a thawing medium. The thawing medium was warmed to 37°C.
Procedure 2) One vial containing cryopreserved cardiomyocytes derived from human iPS cells was warmed for 120 seconds in a water bath at 37°C.
Procedure 3) The cell suspension was transferred from the vial warmed in Procedure 2) into a 15-mL tube containing 10 mL of the thawing medium prepared in Procedure 1). At that time, an operation of homogenizing the cell suspension using a pipetter was not performed.
Procedure 4) The vial in Procedure 3) was washed with 1 mL of the thawing medium in the tube in Procedure 3), and 1 mL of the medium used for the washing was put back in the tube described above.
Procedure 5) Centrifugation of the tube in Procedure 4) was performed at 300 × g for 3 minutes, followed by removing a supernatant.
Procedure 6) The cells precipitated in Procedure 5) were resuspended in 500 µL of the thawing medium, and cell counting was performed using a Countess II FL automatic cell counter manufactured by Thermo Fisher Scientific K.K.
Procedure 7) The cell suspension in Procedure 6) in an amount comprising 3.0 × 10⁶ viable cells was passed through a strainer having an opening of 70 µm, on the basis of the results of the cell counting, the thawing medium was added thereto to achieve a total amount of 5 mL, and the resultant was seeded in the reactor.
Procedure 8) Culture was started at a blade tip speed of 5.5 × 10⁻² m/s (reactor stirring speed of 35 rpm). (This time point is set as "0 hours after the start of the culture".)
Procedure 9) 3 hours after the start of the culture, the presence of precursors having spheroid diameters of 500 µm or more was observed as seen in Figure 2, and the blade tip speed was increased to 6.3 × 10⁻² m/s (reactor stirring speed of 40 rpm). The blade tip speed was increased to 7.1 × 10⁻² m/s (reactor stirring speed of 45 rpm) 6 hours after the start of the culture, the blade tip speed was increased to 8.0 × 10⁻² m/s (reactor stirring speed of 51 rpm) 7 hours after the start of the culture, and the blade tip speed was increased to 8.7 × 10⁻² m/s (reactor stirring speed of 55 rpm) 9 hours after the start of the culture.

### (Example 6)

Procedures 1) to 7) as described in Example 1 were performed, and culture was started at a blade tip speed of 7.9 × 10⁻³ m/s (reactor stirring speed of 5 rpm).

### (Evaluation Example 1: Evaluation of Forms of Cardiomyocyte spheroids)

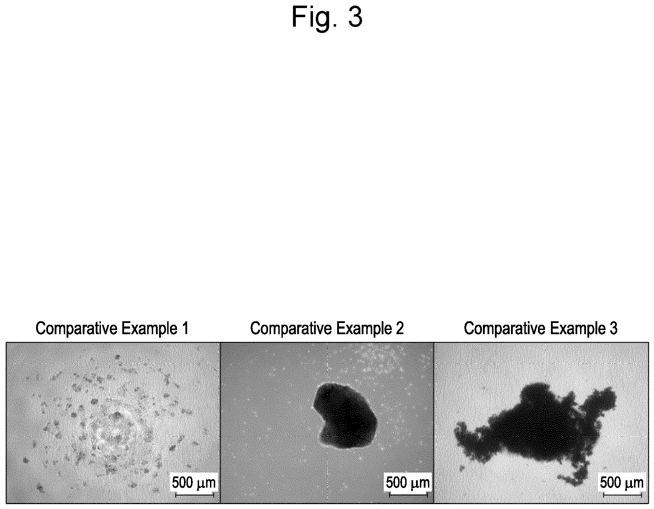

The forms of cells 48 hours after the start of the culture in Comparative Examples 1 to 3 are shown in Figure 3.

In Comparative Example 1, neither production of a cardiomyocyte spheroid nor beating of a cardiomyocyte spheroid was observed by 48 hours after the start of the culture. In the case of static culture, it is expected that time is needed before formation of a cardiomyocyte spheroid because flow of cells is insufficient. In contrast, in Comparative Example 2, which attempted production of a cardiomyocyte spheroid in rotating culture, formation of the cardiomyocyte spheroid was observed; however, over-aggregation of the cardiomyocyte spheroid occurred, and one large cardiomyocyte spheroid (over-aggregated spheroid) was formed. A transplantation needle used in transplantation may be clogged with an over-aggregated spheroid, and cells inside an over-aggregation cannot be supplied with nutrients and cannot excrete waste products to die. Therefore, formation of an over-aggregated spheroid is undesirable. In Comparative Example 3, debris present at the time of thawing was removed in advance by a cell strainer, and therefore, no debris was present in the culture medium just after the seeding; however, generation of new debris was observed 24 hours after the start of the culture. This can be considered to be because dead cells that had passed through the strainer formed the new debris. It was observed that the formed cardiomyocyte spheroid was caught by the debris to over-aggregate 48 hours after the start of the culture. It is considered that, in the rotating culture, the liquid flowed toward the center of the container, and therefore, the cells were accumulated in the center of the container, whereby the new debris was generated.

The results of Comparative Examples 2 and 3 revealed that use of a floating rotating method was not suitable for a technique of producing a cardiomyocyte spheroid.

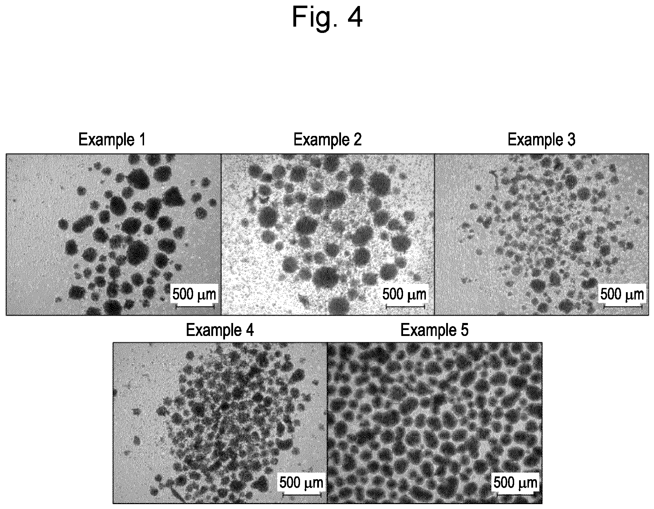

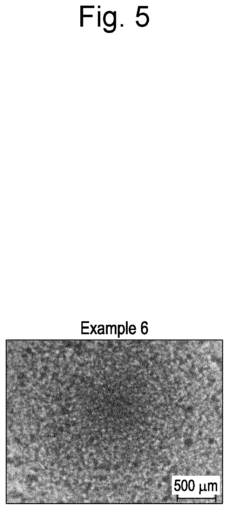

The forms of the cells 48 hours after the start of the culture in Examples 1 to 5, and the forms of the cells 24 hours after the start of the culture in Example 6 are shown in Figures 4 and 5, respectively.

It was observed that cardiomyocyte spheroids were formed 24 hours after the start of the culture in Examples 1 to 5. In Example 6, formation of cardiomyocyte spheroids was not observed, and the cardiomyocytes remained single cells 24 hours after the start of the culture, however, formation of cardiomyocyte spheroids was observed 48 hours after the start of the culture. Moreover, beating cardiomyocyte spheroids were observed 48 hours after the start of the culture in Examples 1 to 4 and 6, and 24 hours after the start of the culture in Example 5. It is considered that, unlike the static culture as in Comparative Example 1, the cells flowed sufficiently in the stirred culture so that frequency of contact between the cells was improved and intercellular junctions were quickly formed, which shortened a period to aggregation and drastically shortened time to start of beating as compared to that in a conventional technology. Moreover, it is considered that, unlike the rotating culture as in Comparative Examples 2 and 3, the cells were prevented from gathering in the center of the culture container in the stirred culture so that generation of debris and an over-aggregated spheroid was suppressed.

### (Evaluation Example 2: Measurement of Sizes of Cardiomyocyte spheroids)

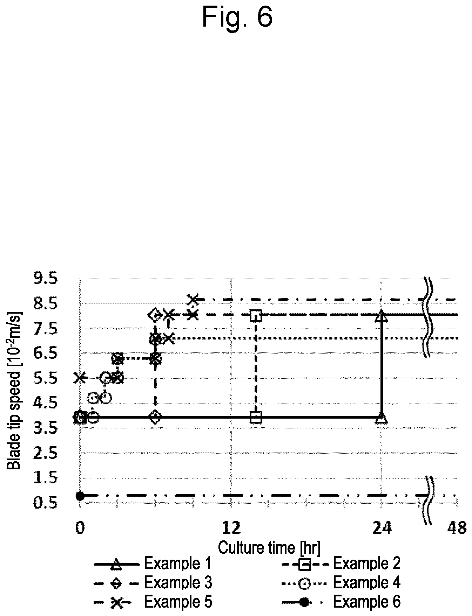

The changes in the blade tip speeds in Examples 1 to 6 are shown in Figure 6.

The sizes of the cardiomyocyte spheroids obtained in Examples 1 to 5 were measured 48 hours after the start of the culture. The results are shown in Table 2. A minimum Feret diameter and a maximum Feret diameter were determined using the measurement function of ZEN lite (free software) from images of cells or cell spheroids captured with a routine inverted microscope Primovert manufactured by ZEISS, and an average value of the minimum Feret diameter and the maximum Feret diameter was calculated as the size of each cell or cell spheroid. A ratio between a minimum Feret diameter and a maximum Feret diameter was calculated as an aspect ratio. The number of cells forming cardiomyocyte spheroids was estimated from the sizes of the cardiomyocyte spheroids, and the percentage of the total number thereof to the number of seeded viable cells was calculated as formation efficiency. The results of Table 2 reveal that the size of a cardiomyocyte spheroid can be freely controlled by changing a blade tip speed in culture, or by adjusting the timing of the change. For example, in Example 4, inhibition of adhesion between the cells due to shearing force was able to be suppressed by reducing the maximum value of the blade tip speed as compared to that in Example 3, and thus, cardiomyocyte spheroids having larger sizes than those in Example 3 could be produced. In addition, in Example 4, the blade tip speed could be maintained such that adhesion between cardiomyocytes was not inhibited while over-aggregation of cardiomyocyte spheroids was prevented, by increasing the blade tip speed in a stepwise manner from the early period of the culture, and thus, cardiomyocyte spheroids having uniform sizes could be obtained in comparison with Example 3. This is demonstrated by the lower percentage of the standard deviation of the sizes of the cell spheroids with respect to the average of the sizes of the cell spheroids in Example 4 as compared to that in Example 3 (Table 2). In Example 5, though cardiomyocyte spheroids were produced using a cell density four times greater than those in Examples 1 to 4, cardiomyocyte spheroids could be obtained without any problems such as over-aggregation.

**[Table 2]**

| | Average size [µm] | Maximum size [µm] | Minimum size [µm] | Standard deviation [µm] | Standard deviation! average size [%] | Average aspect ratio | Formation efficiency [%] |
|---|---|---|---|---|---|---|---|
| Example 1 | 174 | 320 | 50 | 66 | 38 | 0.87 | 25 |
| Example 2 | 171 | 359 | 41 | 73 | 43 | 0.88 | 46 |
| Example 3 | 95 | 192 | 33 | 32 | 34 | 0.83 | 30 |
| Example 4 | 107 | 217 | 33 | 32 | 30 | 0.86 | 50 |
| Example 5 | 186 | 287 | 62 | 44 | 24 | 0.80 | 77 |

### (Evaluation Example 3)

Live/Dead assay of the cardiomyocyte spheroids obtained in Example 4 was performed using Live/Dead Cell Staining Kit II (Promokine), 48 hours after the start of the culture. Observation was performed using BZ-810X (KEYENCE) as a fluorescence microscope. Procedures of the Live/Dead assay were performed according to a protocol included in the kit. In brief, the cardiomyocyte spheroids obtained in Example 4 were dispersed in PBS comprising Calcein-AM and ethidium homodimer III (EthD-III) at concentrations of 2 µM and 4 µM, respectively, and allowed to react for 30 minutes, at room temperature, and under a light-shielding condition.

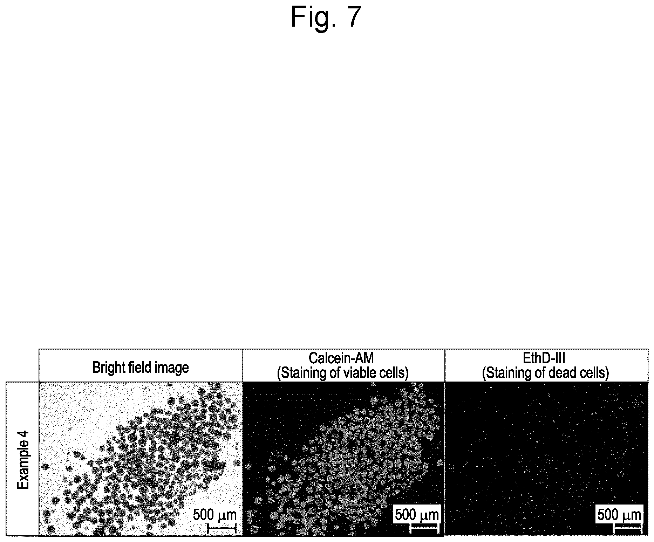

The results of the Live/Dead assay are shown in Figure 7. ImageJ was used for image analysis. An area stained with EthD-III, a dead cell marker, was 4.0% of an area stained with Calcein-AM, a viable cell marker. This means that most of the cardiomyocyte spheroids obtained in Example 4 are formed of viable cells, and suggests that the cardiomyocyte spheroids have very excellent quality.

These Examples demonstrate that a large number of cardiomyocyte spheroids can be very easily produced in a short term by the method of the present invention. Thus, it is expected that the present invention greatly contributes to transplantation therapy and the like.

All publications, patents, and patent applications cited herein are incorporated herein by reference in their entirety.

## Claims

1. A method of producing a cardiomyocyte spheroid, the method comprising a step of culturing cardiomyocytes separated into single cells while stirring liquid in a container in which the cardiomyocytes are suspended, thereby causing aggregation of the cells.

2. The method of producing the cardiomyocyte spheroid according to claim 1, wherein the container has a cell non-adherent inner face.

3. The method of producing the cardiomyocyte spheroid according to claim 1 or 2, wherein a volume of the container is 5 mL or more.

4. The method of producing the cardiomyocyte spheroid according to any one of claims 1 to 3, wherein a speed of the stirring is changed in a stepwise or continuous manner during the culture.

5. The method of producing the cardiomyocyte spheroid according to any one of claims 1 to 4, wherein the speed of the stirring is increased in a stepwise or continuous manner during the culture.

6. The method of producing the cardiomyocyte spheroid according to any one of claims 1 to 5, wherein the cardiomyocytes are derived from a pluripotent stem cell.

7. The method of producing the cardiomyocyte spheroid according to any one of claims 1 to 6, wherein debris derived from the cardiomyocytes is removed in advance using a cell strainer prior to start of the culture of the cardiomyocytes.

8. The method of producing the cardiomyocyte spheroid according to any one of claims 1 to 7, wherein a cell concentration of the cardiomyocytes in the liquid is 1.0 × 10³ cells/mL or more and 1.0 × 10⁸ cells/mL or less.

9. A cardiomyocyte spheroid produced by the method according to any one of claims 1 to 8, wherein an average value of an aspect ratio is 0.60 or more.

10. A cardiomyocyte spheroid produced by the method according to any one of claims 1 to 8, wherein a standard deviation of a size of the cell spheroid is 50% or less of an average value of a size of the cell spheroid.

11. A cardiomyocyte spheroid produced by the method according to any one of claims 1 to 8, wherein an average value of a size of the cell spheroid is 30 µm or more and 2000 µm or less.

12. A cardiomyocyte spheroid produced by the method according to any one of claims 1 to 8, wherein the cardiomyocyte spheroid beats.

13. A cardiomyocyte spheroid produced by the method according to any one of claims 1 to 8, wherein a total number of cardiomyocytes constituting the cardiomyocyte spheroid is 15% or more of a number of viable cells seeded in the container.

14. A cardiomyocyte spheroid produced by the method according to any one of claims 1 to 8, wherein in a case where the cardiomyocyte spheroid is double-stained with Calcein-AM and ethidium homodimer III (EthD-III), an area of a region stained with the EthD-III is 30% or less of an area of a region stained with the Calcein-AM.
